(19) **Europäisches Patentamt / European Patent Office / Office européen des brevets**

(11) **EP 1 479 340 B1**

(12) **EUROPEAN PATENT SPECIFICATION**

(45) Date of publication and mention
of the grant of the patent:
**19.12.2007 Bulletin 2007/51**

(51) Int Cl.:
*A61B 5/053* (2006.01)     *A61B 5/22* (2006.01)

(21) Application number: **04011785.5**

(22) Date of filing: **18.05.2004**

(54) **Apparatus for assessing leg strength of a user**

Verfahren zur Abschätzung der Beinkraft eines Benutzers

Appareil pour évaluer la force musculaire de la jambe d'un utilisateur

(84) Designated Contracting States:
**DE FR GB**

(30) Priority: **21.05.2003 JP 2003143068**

(43) Date of publication of application:
**24.11.2004 Bulletin 2004/48**

(73) Proprietor: **TANITA CORPORATION**
**Tokyo (JP)**

(72) Inventors:
• **Aoyagi, Yukitoshi**
  **Tokyo (JP)**
• **Itagaki, Shuji**
  **c/o Tanita Corporation**
  **Tokyo (JP)**

(74) Representative: **Grünecker, Kinkeldey,**
**Stockmair & Schwanhäusser**
**Anwaltssozietät**
**Maximilianstrasse 58**
**80538 München (DE)**

(56) References cited:
**EP-A- 1 306 051**

• **HARRIS T: "Muscle mass and strength: relation to function in population studies" THE JOURNAL OF NUTRITION, vol. 127, no. 5 Suppl, May 1997 (1997-05), pages 1004S-1006S, XP002296173 UNITED STATES ISSN: 0022-3166**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

**Description**

The present invention relates to a leg strength assessment apparatus for assessing leg strength of a user according to the preamble of claim 1.

Such apparatus is known, for example, from EP-A-1 306 051.

Recently, with the progress of advanced age society, number of vigorous old men and women becomes increased. On the other hand, among those, there are many people who have some apprehensions about so called "bedridden" condition. In such circumstances there have been greater needs in the market for devices and apparatus for assessing about, for example, "What activity can I have?" or "Does my legs have sufficient strength for supporting my own body?" (which is defined, here, as "leg strength").

In the past, in response to such needs in the market, various types of devices and apparatus have been developed and provided. Among those, however, there has been no such apparatus available in the market that is capable of assessing leg strength of a user to some degree, except for the followings: a high precision body fat meter available in the market in the product name "Body Planner" (see Non-Patent Document 1); and a health condition assessment and display apparatus, as disclosed in Patent Laid-Open No. WO 01/015600 (see Patent Document 1).

In particular, the apparatus available in the product name "Body Planner", as described above, is configured to estimate muscle mass for each of body parts of a user depending on the bioelectrical impedance ("BI") thereof and the like and to display the result of estimation with any one of several 'levels''. Furthermore, the health condition assessment and display apparatus, as described above, is proposed to provide capability of estimating muscle mass in the lower limbs (i.e. legs) of a user depending on the bioelectrical impedance thereof and the like and of producing an index for result for treatment by exercise, as compared to fat mass.

The documents associated with the present invention are:

Non-Patent Document 1:
High precision body fat meter, "Body Planner", made by Yamato Seiko K. K., Internet URL: http://www.yamato-scale.co.jp/en/product/detail.php; and

Patent Document 1:
Patent Laid-Open No. WO 01/015600, Official Gazette (page 25).

However, the high precision body fat meter, "Body Planner", as described above, is defective in that muscle mass is simply replaced with the "level", which alone is insufficient for assessment of leg strength. In particular, even if the lower limbs muscle level is higher (or muscle mass is greater), legs cannot support such a body as having a greater mass of tissue other than muscle (e.g. fat mass), that is to say, the leg strength is not enough. In addition, "criterion" for the level is not clearly defined, and consequently, relative comparison between body parts can only be done. As the result, the user can't objectively grasp own leg strength and can't easily understand it.

Furthermore, as described above, the health condition assessment and display apparatus produces the index as compared to fat mass. However, in view of the fact that the body supported by the legs is not constituted only by fat (especially, fat is lighter in unit mass, as compared to muscle), it is hard to say that the index is suitable as the measure of the leg strength. Accordingly, provision of such index does not contribute to proposal for concrete configuration of devices and apparatus that can meet the need in the market, as described above.

In view of the above, it is an object of the present invention to solve the problems in the prior art, as described above, and to provide an improved apparatus for assessing leg strength of a user, that can sufficiently fulfill the need in the market, as described above.

Summary of the Invention:

To attain such object the present invention provides, in one aspect thereof, the apparatus for assessing leg strength of a user according to claim 1, the apparatus comprising: a leg strength information acquisition unit; a reference information storage unit; and a leg strength assessment unit, wherein

said leg strength information acquisition unit acquires leg strength information representing lower limbs muscle mass relative to at least one of body weight and height of the user,
said reference information storage unit stores the predetermined reference information in advance, and
said leg strength assessment unit assesses leg strength of the user by comparing the leg strength information acquired by said leg strength information acquisition unit with the predetermined reference information stored in said reference information storage unit , wherein
said predetermined reference information includes an average of leg strength information for a plurality of specific persons.

According to another embodiment of the present invention said specific persons are young people who are in good health.

According to further embodiment of the present invention said specific persons are same aged people as the user.

According to yet further embodiment of the present invention said leg strength assessment unit indicates the leg strength of the user by comparing the leg strength information of the user with said predetermined reference information, assuming that said predetermined reference information has the score of 100 points.

According to yet further embodiment of the present invention said leg strength assessment unit indicates the leg strength of the user by comparing the leg strength information of the user on a graph including said predetermined reference information.

According to yet further embodiment of the present invention said leg strength assessment unit indicates the leg strength of the user by comparing the leg strength information of the user on zones of a graph "Body Weight or Body Mass Index (BMI) - Leg Strength Information" including said predetermined reference information.

According to yet further embodiment of the present invention said apparatus further comprises an age information acquisition unit that acquires age information of the user, and said leg strength assessment unit indicates the leg strength of the user by comparing the leg strength information of the user on zones of a graph "Age-Leg Strength Information" including said predetermined reference information.

According to yet further embodiment of the present invention said reference information storage unit stores a second reference information associated with said predetermined reference information in advance, and said leg strength assessment unit indicates the leg strength of the user by comparing the leg strength information of the user on a graph including said predetermined reference information and said second reference information.

According to yet further embodiment of the present invention said leg strength information acquisition unit includes: a body information acquisition unit; a bioelectrical impedance (BI) measurement unit; a lower limbs muscle mass calculation unit; and a leg strength information calculation unit, wherein

said body information acquisition unit acquires body information including at least height and body weight of the user,
said BI measurement unit measures bioelectrical impedance between specific body parts of the user,
said lower limbs muscle mass calculation unit calculates lower limbs muscle mass based on the body information acquired by said body information acquisition unit and the bioelectrical impedance measured by said BI measurement unit, and
said leg strength information calculation unit calculates leg strength information of the user based on the body information acquired by said body information acquisition unit and the lower limbs muscle mass calculated by said lower limbs muscle mass calculation unit.

According to yet further embodiment of the present invention said leg strength information calculation unit calculates the leg strength information according to an equation of "lower limbs muscle mass / body weight".

According to yet further embodiment of the present invention said leg strength information calculation unit calculates the leg strength information according to an equation of "lower limbs muscle mass / height$^2$".

According to yet further embodiment of the present invention said body information acquisition unit acquires the height of the user by entering or measuring the same, and acquires the body weight of the user by measuring the same, and said BI measurement unit measures the bioelectrical impedance between both feet of the user.

According to yet further embodiment of the present invention said BI measurement unit measures the bioelectrical impedance between both hands and feet of the user.

According to yet further embodiment of the present invention said user is an advanced age person.

The present invention provides, in another aspect thereof, a computer readable record medium having a program recorded thereon for running a computer as an apparatus for assessing leg strength of a user, as described above.

The present invention provides, in further aspect thereof, a program for running a computer as an apparatus for assessing leg strength of a user, as described above.

Brief Description of the Drawings:

The present invention will, now, be described in more detail with reference to the accompanying drawings, in which:

Fig. 1 is a perspective view of a leg strength assessment apparatus for assessing leg strength of a user according to one embodiment of the present invention;
Fig. 2 is an electrical block diagram of the leg strength assessment apparatus in Fig. 1;
Fig. 3 is a flow chart for illustrating operation of the leg strength assessment apparatus in Fig. 1;
Fig. 4 shows one example of display screen of a display unit in the leg strength assessment apparatus in Fig. 1;
Fig. 5 shows another example of display screen of a display unit in the leg strength assessment apparatus in Fig. 1;
Fig. 6 shows further example of display screen of a display unit in the leg strength assessment apparatus in Fig. 1;
Fig. 7 shows yet further example of display screen of a display unit in the leg strength assessment apparatus in Fig. 1; and
Fig. 8 shows yet further example of display screen of a display unit in the leg strength assessment apparatus in Fig. 1.

Description of the Preferred Embodiments:

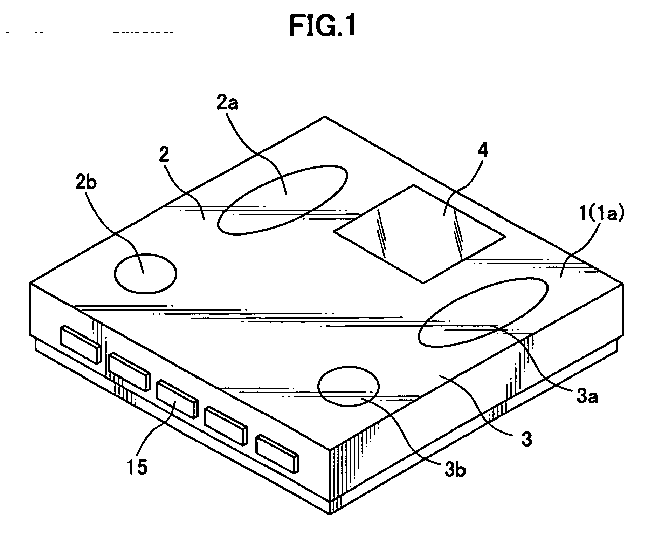

Fig. 1 is a perspective view of a leg strength assessment apparatus 1 for assessing leg strength of a user according to one embodiment of the present invention. The leg strength assessment apparatus 1 in this embodiment generally consists of a prior art weight meter having a bioelectrical impedance ("BI") measurement function added thereto. In particular, the apparatus 1 includes a platform 1a on which a user stands for measuring his/her body weight, and left and right electrode sections 2, 3 provided on the platform for contacting to left and right soles of the user. The electrode sections 2, 3 include current supplying electrodes 2a, 3a and voltage measurement electrodes 2b, 3b. (It is called "four-electrode system".) A display and input unit 4 is provided on the top of the apparatus 1. In addition, a plurality of foot switches 15 are provided at the front side of the apparatus 1 for performing functions as described later.

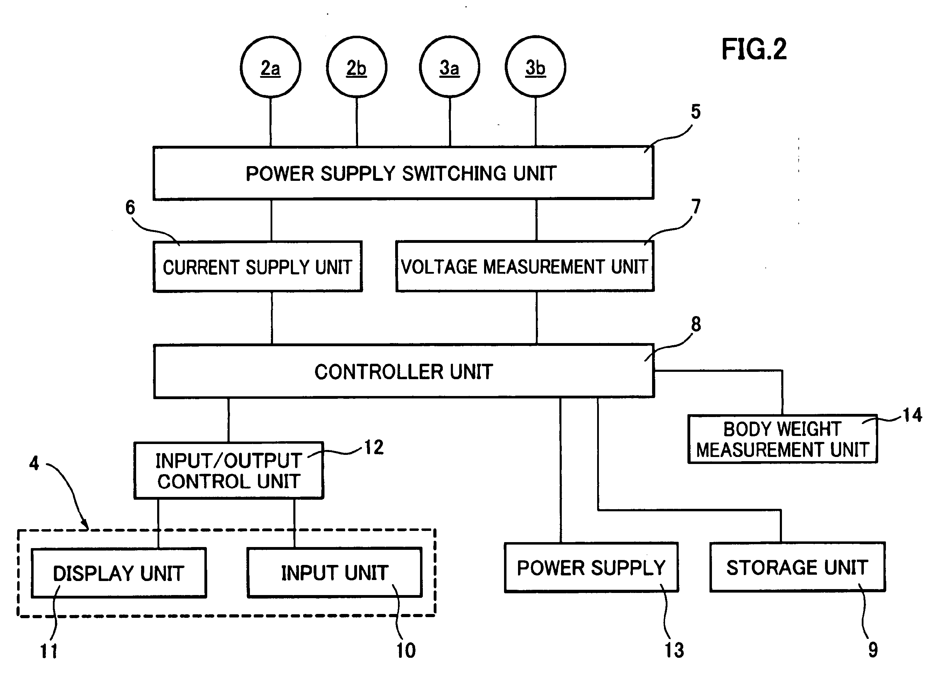

Fig. 2 is an electrical block diagram of the leg strength assessment apparatus 1. As shown in Fig. 2, the four electrodes 2a, 2b, 3a, 3b for contacting to left and right soles of the user are connected to a power supply switching unit 5. The power supply switching unit 5 is connected to a controller unit 8 via a current supply unit 6 and a voltage measurement unit 7. The controller unit 8 includes a microcomputer and is connected to a storage unit 9 for storing various types of data therein. The display and input unit 4 consists of an input unit 10 and a display unit 11 which are electrically independent circuits. The display and input unit 4 is connected to the controller unit 8 via an input/output control unit 12 which is provided for controlling the input unit 10 and the display unit 11. The leg strength assessment apparatus 1 further includes a power supply unit 13 for supplying power to the controller unit 8 and other units. The controller unit 8 controls operation of a body weight measurement unit 14.

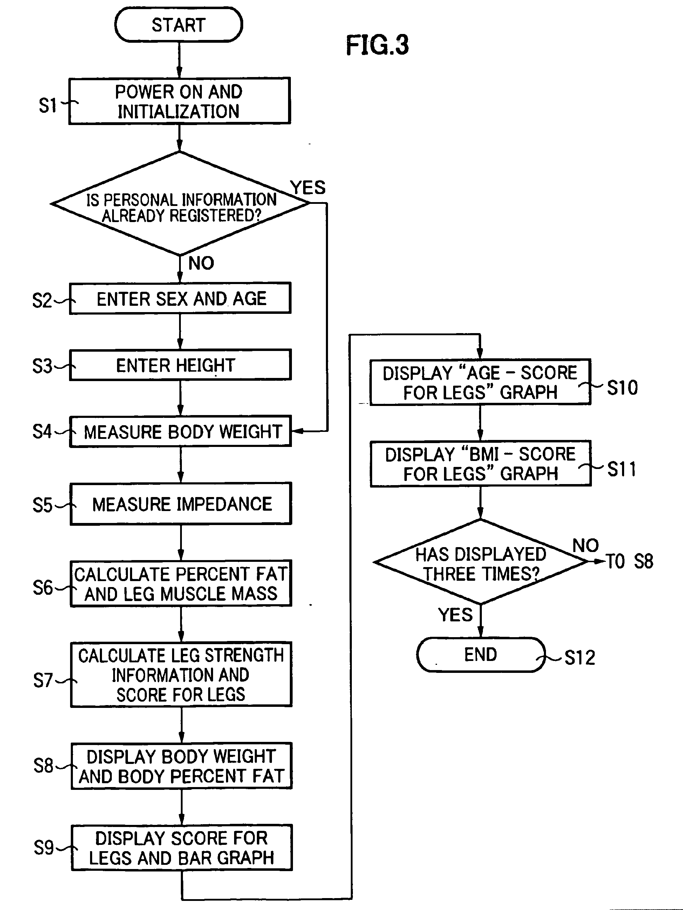

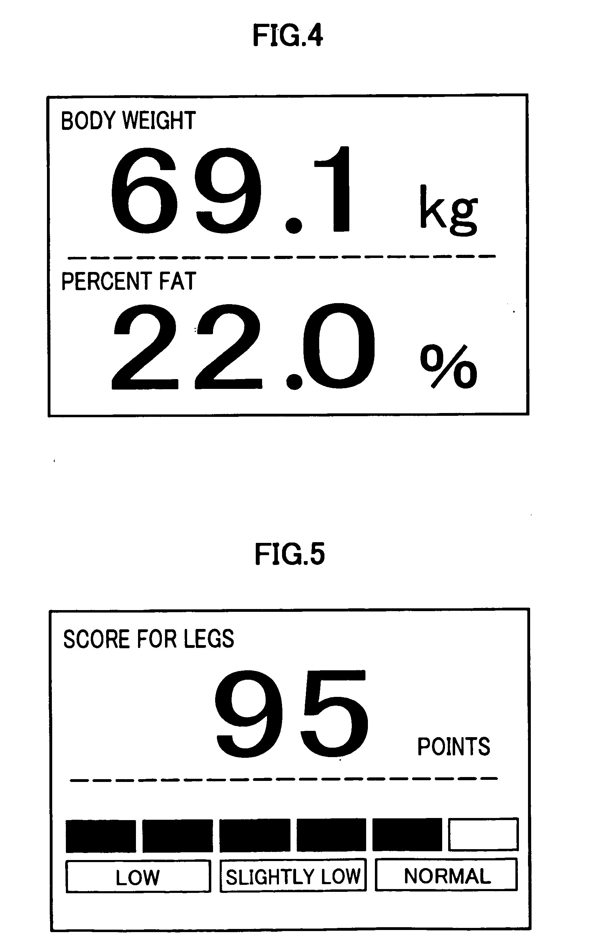

Now, operation of the leg strength assessment apparatus 1 in this embodiment will be described with reference to a flow chart in Fig. 3 and examples of display screen in Figs. 4 to 8. First of all, a user turns ON one of the foot switches 15 to initialize the entire electrical system at step S1. In this regard, a plurality of switches 15 is provided each for each of users, which makes possible to simply retrieve the initial input value. At step S2 the user enters sex and age data. At this time, some message or mark (not shown) for prompting the user to enter sex and age data is displayed on the display unit 11 of the display and input unit 4. Then, the routine proceeds to step S3 where the user enters height data. An up key or a down key on the display and input unit 4 is used to enter the height data. For example, assuming that height of the user is 170 cm and the default value for height is 120 cm, then the up key may repeatedly be depressed 50 times or it may be kept depressed for more than the fixed time period to provide continuous numeral input until height of 170 cm is reached. The initial data input operation is terminated up to step S3. Alternatively, entering of height data may be performed by measuring the height of the user with some electronic measurement device and the like. The body information including sex, age, height, etc. is stored in the storage unit 9 under the control of the controller unit 8.

Then, at step S4, the leg strength assessment apparatus 1 enters body weight measurement mode where number "00" and some message for prompting the user to stand on the platform 1a with his or her feet aligned to the electrode sections 2, 3 are displayed on the display unit 11 of the display and input unit 4, together with another message for prompting the user to maintain his or her posture during the measurement. When the user stands on the platform 1a the body weight of the user is measured and the routine proceeds to step S5 where bioelectrical impedance of the user is measured. The body weight of the user that has separately been measured may be entered to the apparatus. The bioelectrical impedance value is calculated based on electric current value and electric voltage value from the current supplying unit 6 and the voltage measurement unit 7 under the control of the controller unit 8. The body weight and the bioelectrical impedance are stored in the storage unit 9 under the control of the controller unit 8.

Then, at step S6, percent fat and lower limbs muscle mass of the user are calculated. In particular, the percent fat of the user is calculated, under the control of the controller unit 8, depending on the sex data acquired at step S2 and stored in the storage unit 9, the height data acquired at step S3 and stored in the storage unit 9, the body weight measured at step S4 and stored in the storage unit 9, and the bioelectrical impedance data measured at step S5 and stored in the storage unit 9. The lower limbs muscle mass ("LMM") is calculated, under the control of the controller unit 8, depending on the height data ("Ht") acquired at step S3 and stored in the storage unit 9, the body weight ("Wt") measured at step S4 and stored in the storage unit 9, and the bioelectrical impedance ("Z") data measured at step S5 and stored in the storage unit 9, using the following equation, for example. (Some correction term using age or sex data as the variable may be added to the equation.)

$$LMM = a \times Ht^2 / Z + b \times Wt + c$$

where "a", "b" and "c" are constant.
The calculated values for percent fat and lower limbs muscle mass are stored in the storage unit 9 under the control of the controller unit 8.

Next, at step S7, the calculated lower limbs muscle mass is processed with the body weight or height data to produce an index which is then converted into score or number of points. In particular, the calculated lower limbs muscle mass is divided by the body weight to produce an index value (or leg strength information) representing the leg strength of the user. Alternatively, the index value may be produced by dividing the calculated lower limbs muscle mass by square of height. Then, the index value is converted into score or number of points for legs by comparing it with the reference information which is the average for index values of young people (in this embodiment the average for total 390 male and female persons at the ages of 20 to 25 years old) and which is assumed to be at the score of 100 points. The score or number of points for legs is considered to represent the strength for a lower half body of the user. The reference information has been stored in the storage unit 9 in advance. The number of points for legs may be assessed using the average for the same aged people as the user (in another embodiment).

Although the predetermined reference information is used for comparison in the above mentioned embodiment of the present invention, various types of reference information may be used. For example, the average for leg strength information of several specific people may be used as the reference information. The word "specific people", as used here, may include those suitably selected by age, specific age group, sex, body weight, occupation (common people or some sport players), etc. For example, in the example as described above, the specific people selected are young people who are in good health. In such example, the leg strength information of the user is compared with the average for that of young people in good health who are considered to have highest activity (e.g. at the ages from later half of 10's to 30's). Accordingly, it is possible to objectively assess the leg strength of the user in view of active daily life that is desired for him or her. Alternatively, the same aged people as the user may be selected for the specific people, as described above. In this case, the leg strength information of the user is compared with the average for that of the sage aged people, and therefore, it is possible to objectively assess his or her leg strength while being encouraged by comparison to the same aged people.

Then, at step S8, the body weight is displayed on the upper portion of the display screen and the percent fat is displayed on the lower portion thereof in the display unit 11 of the display and input unit 4 (see Fig. 4). Thereafter, at step S9, the score for legs and the corresponding bar graph are displayed (see Fig. 5). In this embodiment the bar graph is divided into total six blocks which are individually turned ON. In particular, at the score of not less than 120 points all blocks are turned ON; at the score from not less than 90 to less than 120 points five blocks are turned ON; at the score from not less than 85 to less than 90 four blocks are turned ON; at the score from not less than 80 to less than 85 three blocks are turned ON; at the score from not less than 50 to less than 80 two blocks are turned ON; and at the score of less than 50 points one block is turned ON. Comments for assessment such as "Normal", "Slightly Low" and "Low" are provided corresponding to each two blocks, respectively.

Converting the leg strength into the score or number of points and displaying the leg strength by bar graph including the reference information at the score of 100 points in such manner makes possible to easily assess whether the score for legs of the user is enough or not. More precisely, the blocks of the bar graph and the comments act as second reference information relative to the reference information (at the score of 100 points), which makes the assessment more easier.

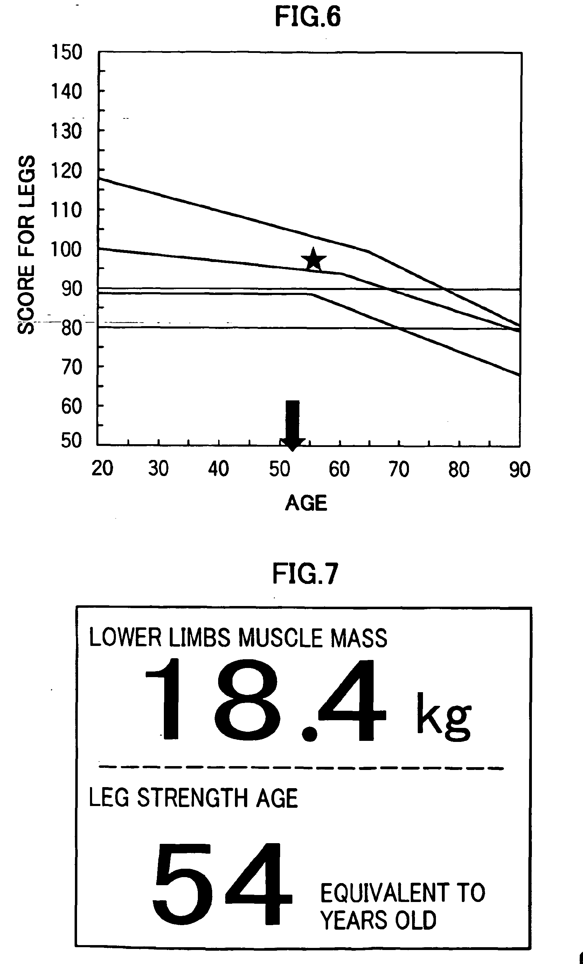

Next, at step S10, a graph showing the relation between the score for legs and the age is displayed (see Fig. 6). In the graph of Fig. 6 the age is plotted on the horizontal axis and the score for legs is plotted on the vertical axis. Alternatively, instead of the score for legs, the index value for leg strength information (i.e. the value of lower limbs muscle mass divided by weight or squire of height) may be plotted on the vertical axis. A curve at the middle portion in Fig. 6 is prepared by connecting the average values (i.e. reference values) for the scores for legs (or index values) that have been calculated for people of each age in order to show how the score for legs is changed with the age. In addition, curves above and below the curve at the middle portion, as described above, represent proper deviation range at each age (or second reference value) that is provided by some statistical process such as standard deviation ("SD") or percentile relative to the average for people of each age. Furthermore, the graph has two straight lines in parallel to the horizontal axis: the upper one is at the value (the second reference value: -1SD) that is resulted from subtracting the standard deviation from the average value (reference value at the score of 100 points) for a group of young people; and the lower one is at the value (-2SD) that is resulted from subtracting twice the standard deviation therefrom. The zone over "-1SD" line means that the leg strength present therein is "Good" or "Normal"; the zone between "-1SD" and "-2SD" lines means that "Little attention is necessary to pay; and the zone under "-2SD" line means that "Attention is necessary to pay". The zones may be identified by color of blue, yellow and red. A star mark (*) in the graph indicates the result of measurement for a user, and an arrow (↓) on the horizontal axis indicates the age of the user whose score for legs corresponds to the average for people at that age. In other words, it indicates "leg strength age" of the user in view of lower limbs muscle mass, not the true age of the user. Such leg strength age may be displayed together with the calculated lower limbs muscle mass, as shown in Fig. 7. It is noted that the second reference information has also been stored in the storage unit 9 in advance. The second reference information is not limited to those described above, but it may be defined as 90%, 80%, etc. of the reference information.

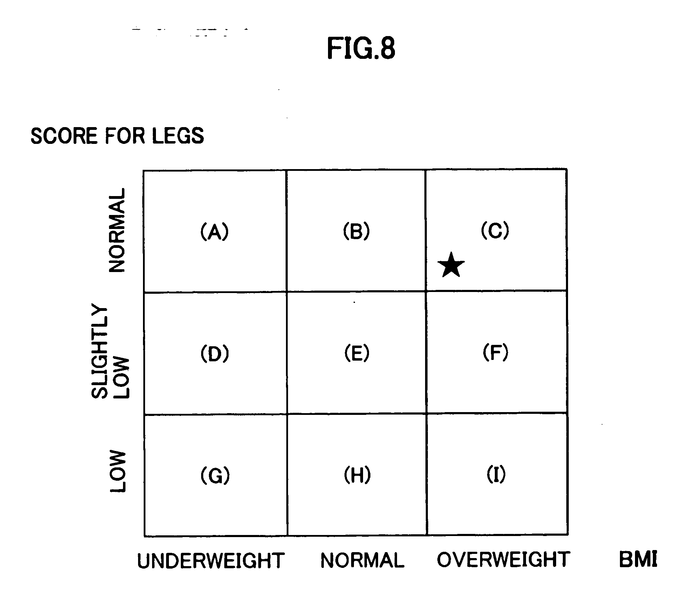

Then, at step S11, a cross table showing the relation between the score for legs and the body mass index ("BMI" = body weight / height$^2$) is displayed (see Fig. 8). In Fig. 8 the score for legs is shown on the vertical axis with three rows: "Normal"; "Slightly Low"; and "Low", in the same manner as the bar graph in Fig. 5. On the other hand, "BMI" of the user is shown on the horizontal axis with three columns: "Overweight"; "Normal"; and "Underweight" (Threshold between adjacent columns: "BMI" = 25 & 18.5). The threshold between adjacent rows on the vertical axis may be at "- 1SD" and "-2SD" as used in Fig. 6. Furthermore, "BMI" on the horizontal axis may be replaced with body weight. A star mark (★) indicates the area where the measurement value for the user is present. The cross table has a plurality of zones (A- I) separated by the threshold, as described above. The zones may be identified by color or may be provided with some comment. For example, the zones "A", "B", "D" and "E" may be provided with the comment "Normal", the zones "C", "F" and "G" may be provided with "Little attention is necessary to pay, and the zones "H" and "I" may be provided with "Attention is necessary to pay".

After repeating steps S8 to S11 predetermined number of times (for example, three times) the power supply is automatically turned OFF (at step S12).

In the above embodiment, improvement in a weight meter having a four-electrode "BI" meter provided therein for measurement of "BI" between both feet of a user has been described. However, any modification, as described above with the embodiment, can, of course, be made, and "BI" measurement with eight-electrode system may be performed between both hands and both feet (in this case measurement of muscle mass can be performed for each of left and right legs so that more precise assessment for leg strength can be conducted). Or alternatively, "BI" measurement with four-electrode system (e.g. card type and portable type) may be performed between both hands. Such modified embodiments can easily be implemented by, for example, adjusting the constant and the like in the calculation formula for calculating the lower limbs muscle mass (LMM), as described above.

Acquisition of information for lower limbs muscle mass relative to body weight or height is not limited to "BI" method, as described above. It may, of course, be performed with "MRI" (Magnetic Resonance Imaging), CT (Computed Tomography) and "DEXA" (Dual Energy X-ray Absorptiometry).

Furthermore, the present invention is not limited to the unit product in the above described embodiment, but may be implemented in the form of program software executable on general purpose computers.

It is apparent from the foregoing that a leg strength assessment apparatus according to the present invention can objectively assesses leg strength of a user with dear index and criterion. Moreover, the apparatus can easily assess the leg strength of the user by simple display of the relation between the index and the criterion.

**Claims**

1. An apparatus (1) for assessing leg strength of a user, comprising:

   a leg strength information acquisition unit (2,3,4,5,6,7,8) ;
   a reference information storage unit (9); and
   a leg strength assessment unit (2,3,4,5,6,7,8), wherein
   said reference information storage unit (9) stores predetermined reference information in advance
   **characterised in that**
   said leg strength information acquisition unit acquires leg strength information representing lower limbs muscle mass (LMM) relative to at least one of body weight (Wt) and height (Ht) of the user;
   said leg strength assessment unit assesses leg strength of the user by comparing the leg strength information acquired by said leg strength information acquisition unit (2,3,4,5,6,7,8) with the predetermined reference information stored in said reference information storage unit ; and
   the predetermined reference information is an average of leg strength information for a plurality of specific persons.

2. An apparatus (1) for assessing leg strength of a user according to claim 1 in which said specific persons are young people who are in good health.

3. An apparatus (1) for assessing leg strength of a user according to claim 1 in which said specific persons are same aged people as the user.

4. An apparatus (1) for assessing leg strength of a user according to any one of claims 1 to 3 in which said leg strength assessment unit indicates the leg strength of the user by comparing the leg strength information of the user with said predetermined reference information, assuming that said predetermined reference information has the score of 100 points.

5. An apparatus (1) for assessing leg strength of a user according to any one of claims 1 to 4 in which said leg strength assessment unit indicates the leg strength of the user by comparing the leg strength information of the user on a graph including said predetermined reference information.

6. An apparatus (1) for assessing leg strength of a user according to claim 5 in which said leg strength assessment unit indicates the leg strength of the user by comparing the leg strength information of the user on zones of a graph "Body Weight (Wt) or Body Mass Index (BMI)-Leg Strength Information" including said predetermined reference information.

7. An apparatus (1) for assessing leg strength of a user according to claim 5 in which it further comprises an age information acquisition unit (4) that acquires age information of the user, and said leg strength assessment unit indicates the leg strength of the user by comparing the leg strength information of the user on zones of a graph "Age-Leg Strength Information" including said predetermined reference information.

8. An apparatus (1) for assessing leg strength of a user according to any one of claims 5 to 7 in which said reference information storage unit (9) stores a second reference information associated with said predetermined reference information in advance, and said leg strength assessment unit indicates the leg strength of the user by comparing the leg strength information of the user on a graph including said predetermined reference information and said second reference information.

9. An apparatus (1) for assessing leg strength of a user according to any one of claims 1 to 8 in which said leg strength information acquisition unit (2,3,4,5, 6,7,8,14) includes:

a body information acquisition unit (4,14);
a bioelectrical impedance (BI) measurement unit (2,3,5,6,7);
a lower limbs muscle mass (LMM) calculation unit (8); and
a leg strength information calculation unit (8), wherein
said body information acquisition unit (9,14) acquires body information including at least height (Ht) and body weight (Wt) of the user,
said BI measurement unit (2,3,5,6,7) measures bioelectrical impedance between specific body parts of the user,
said lower limbs muscle mass (LMM) calculation unit (8) calculates lower limbs muscle mass (LMM) based on the body information acquired by said body information acquisition unit (4,14) and the bioelectrical impedance measured by said BI measurement unit (2,3,5,6,7), and
said leg strength information calculation unit (8) calculates leg strength information of the user based on the body information acquired by said body information acquisition unit (4,14) and the lower limbs muscle mass (LMM) calculated by said lower limbs muscle mass (LMM) calculation unit (8).

10. An apparatus for assessing leg strength of a user according to claim 9 in which said leg strength information calculation unit (8) calculates the leg strength information according to an equation of "lower limbs muscle mass (LMM) /body weight (Wt)".

11. An apparatus (1) for assessing leg strength of a user according to claim 9 in which said leg strength information calculation unit calculates the leg strength information according to an equation of "lower limbs muscle mass (LMM) /height$^2$ (Ht$^2$)".

12. An apparatus (1) for assessing leg strength of a user according to any one of claims 9 to 11 in which said body information acquisition unit (4,14) acquires the height (Ht) of the user by entering or measuring the same, and acquires the body weight (Wt) of the user by measuring the same, and said BI measurement unit (2,3,5,6,7) measures the bioelectrical impedance between both feet of the user.

13. An apparatus (1) for assessing leg strength of a user according to any one of claims 9 to 11 in which said BI measurement unit (2,3,5,6,7); measures the bioelectrical impedance between both hands and feet of the user.

14. An apparatus (1) for assessing leg strength of a user according to any one of claims 1 to 13 in which said user under test is an advanced age person.

15. A computer readable record medium having a program recorded thereon for running a computer as an apparatus (1) for assessing leg strength of a user according to any one of claims 1 to 14.

16. A program for running a computer as an apparatus (1) for assessing leg strength of a user according to any one of claims 1 to 14.

**Patentansprüche**

1. Eine Vorrichtung (1) zur Bewertung einer Beinkraft eines Benutzers, umfassend:

eine Einheit (2, 3, 4, 5, 6, 7, 8) zur Beschaffung einer Information über die Beinkraft;
eine Einheit (9) zur Speicherung einer Referenzinformation; und
eine Einheit (2, 3, 4, 5, 6, 7, 8) zur Bewertung einer Beinkraft, wobei die besagte Einheit (9) zur Speicherung der Referenzinformation die vorgegebene Referenzinformation im voraus speichert,
**dadurch gekennzeichnet, dass**
die besagte Einheit zur Beschaffung einer Information über die Beinkraft eine Information über die Beinkraft beschafft, die eine Muskelmasse der unteren Gliedmaßen (LMM) im Verhältnis zu zumindest einem Wert des Körpergewichts (Wt) und der Größe (Ht) des Benutzers repräsentiert;

wobei die besagte Einheit zur Bewertung der Beinkraft die Beinkraft des Benutzers bewertet, durch Vergleichen der Information über die Beinkraft, die durch die besagte Einheit (2, 3, 4, 5, 6, 7, 8) zur Beschaffung einer Information über die Beinkraft beschafft wurde mit der vorgegebenen Referenzinformation, die in der besagten Einheit zur Speicherung der Referenzinformation gespeichert wurde; und wobei die vorgegebene Referenzinformation ein Durchschnittswert einer Information über die Beinkraft für eine Vielzahl von spezifischen Personen ist.

2. Eine Vorrichtung (1) zur Bewertung einer Beinkraft eines Benutzers im Einklang mit Anspruch 1, wobei die besagten spezifischen Personen junge Leute sind, die sich in einem guten Gesundheitszustand befinden.

3. Eine Vorrichtung (1) zur Bewertung einer Beinkraft eines Benutzers im Einklang mit Anspruch 1, wobei die besagten spezifischen Personen Leute derselben Altersgruppe wie der Benutzer sind.

4. Eine Vorrichtung (1) zur Bewertung einer Beinkraft eines Benutzers im Einklang mit einem der Ansprüche 1 bis 3, wobei die besagte Einheit zur Bewertung der Beinkraft die Beinkraft des Benutzers anzeigt, durch Vergleichen der Information über die Beinkraft des Benutzers mit der besagten vorgegebenen Referenzinformation, unter der Annahme, dass die besagte vorgegebene Referenzinformation den Wert von 100 Punkten hat.

5. Eine Vorrichtung (1) zur Bewertung der Beinkraft eines Benutzers im Einklang mit einem der Ansprüche 1 bis 4, wobei die besagte Einheit zur Bewertung der Beinkraft die Beinkraft des Benutzers anzeigt, durch Vergleichen der Information über die Beinkraft des Benutzers an einem Graph, der die besagte vorgegebene Referenzinformation umfasst.

6. Eine Vorrichtung (1) zur Bewertung der Beinkraft eines Benutzers im Einklang mit Anspruch 5, wobei die besagte Einheit zur Bewertung der Beinkraft die Beinkraft des Benutzers anzeigt, durch Vergleichen der Information über die Beinkraft des Benutzers an Bereichen eines Graphs "Körpergewicht (Wt) oder Body Mass Index (BMI) - Information über die Beinkraft", der die besagte vorgegebene Referenzinformation umfasst.

7. Eine Vorrichtung (1) zur Bewertung der Beinkraft eines Benutzers im Einklang mit Anspruch 5, wobei die Vorrichtung (1) ferner eine Einheit (4) zur Beschaffung einer Information über das Alter umfasst, welche eine Information über das Alter des Benutzers beschafft, wobei die besagte Einheit zur Bewertung der Beinkraft die Beinkraft des Benutzers anzeigt, durch Vergleichen der Information über die Beinkraft des Benutzers an Bereichen eines Graphs "Alter - Information über die Beinkraft", der die besagte vorgegebene Referenzinformation umfasst.

8. Eine Vorrichtung (1) zur Bewertung der Beinkraft eines Benutzers im Einklang mit einem der Ansprüche 5 bis 7, wobei die besagte Einheit (9) zur Speicherung einer Referenzinformation eine zweite Referenzinformation im voraus abspeichert, die der besagten vorgegebenen Referenzinformation zugeordnet ist, wobei die besagte Einheit zur Bewertung der Beinkraft die Beinkraft des Benutzers anzeigt, durch Vergleichen der Information über die Beinkraft des Benutzers an einem Graph, der die besagte vorgegebene Referenzinformation und die besagte zweite Referenzinformation umfasst.

9. Eine Vorrichtung (1) zur Bewertung der Beinkraft eines Benutzers im Einklang mit einem der Ansprüche 1 bis 8, wobei die besagte Einheit (2, 3, 4, 5, 6, 7, 8, 14) zur Beschaffung einer Information über die Beinkraft umfasst:

   eine Einheit (4, 14) zur Beschaffung einer Information über den Körper;
   eine Einheit (2, 3, 5, 6, 7) zur Messung einer bioelektrischen Impedanz (BI);
   eine Einheit (8) zur Berechnung einer Muskelmasse der unteren Gliedmaßen (LMM); und
   eine Einheit (8) zur Berechnung einer Information über die Beinkraft, wobei
   die besagte Einheit (9, 14) zur Beschaffung einer Information über den Körper eine Information über den Körper, umfassend zumindest die Größe (Ht) und das Körpergewicht (Wt) des Benutzers, beschafft,

   wobei die besagte Einheit (2, 3, 5, 6, 7) zur Messung der BI die bioelektrische Impedanz zwischen spezifischen Körperteilen des Benutzers misst, wobei die besagte Einheit (8) zur Berechnung der Muskelmasse der unteren Gliedmaßen (LMM) die Muskelmasse der unteren Gliedmaßen (LMM) berechnet, basierend auf der Information über den Körper, die durch die besagte Einheit (4, 14) zur Beschaffung der Information über den Körper beschafft wurde und die bioelektrische Impedanz, die von der besagten Einheit (2, 3, 5, 6, 7) zur Messung der BI gemessen wurde, und wobei die besagte Einheit (8) zur Berechnung einer Information über die Beinkraft die Information über die Beinkraft des Benutzers berechnet, basierend auf der Information über den Körper, die von der besagten Einheit (4, 14) zur Beschaffung der Information über den Körper beschafft wurde und die Muskelmasse der unteren Gliedmaßen (LMM), die von der besagten Einheit (8) zur Berechnung der Muskelmasse der unteren Gliedmaße (LMM) berechnet wurde.

10. Eine Vorrichtung zur Bewertung der Beinkraft eines Benutzers im Einklang mit Anspruch 9, wobei die besagte Einheit (8) zur Berechnung der Information über die Beinkraft die Information über die Beinkraft im Einklang mit einer Gleichung von "Muskelmasse der unteren Gliedmaßen (LMM) / Körpergewicht (Wt)" berechnet.

11. Eine Vorrichtung (1) zur Bewertung einer Beinkraft eines Benutzers im Einklang mit Anspruch 9, wobei die besagte Einheit zur Berechnung einer Information über die Beinkraft die Information über die Beinkraft in Einklang mit einer Gleichung "Muskelmasse der unteren Gliedmaßen (LMM) / Größe$^2$ (Ht$^2$)" berechnet.

12. Eine Vorrichtung (1) zur Bewertung der Beinkraft eines Benutzers im Einklang mit einem der Ansprüche 9 bis 11, wobei die besagte Einheit (4, 14) zur Beschaffung einer Information über den Körper die Größe (Ht) des Benutzers durch Eingabe oder Messung derselben beschafft, und das Körpergewicht (Wt) des Benutzers durch Messung desselben beschafft, wobei die besagte Einheit (2, 3, 5, 6, 7) zur Messung der BI die bioelektrische Impedanz zwischen beiden Füßen des Benutzers misst.

13. Eine Vorrichtung (1) zur Bewertung der Beinkraft eines Benutzers im Einklang mit einem der Ansprüche 9 bis 11, wobei die besagte Einheit (2, 3, 5, 6, 7) zur Messung der BI die bioelektrische Impedanz zwischen beiden Händen und Füßen des Benutzers misst.

14. Eine Vorrichtung (1) zur Bewertung der Beinkraft eines Benutzers im Einklang mit einem der Ansprüche 1 bis 13, wobei der besagte, dem Test unterzogene Benutzer, eine Person eines fortgeschrittenen Alters ist.

15. Ein Computer - lesbares Aufnahmemedium mit einem darauf abgespeicherten Programm zum Abspielen auf einem Computer als eine Vorrichtung (1) zur Bewertung einer Beinkraft eines Benutzers im Einklang mit einem der Ansprüche 1 bis 14.

16. Ein Programm zum Abspielen auf einem Computer als eine Vorrichtung (1) zur Bewertung einer Beinkraft eines Benutzers im Einklang mit einem der Ansprüche 1 bis 14.

**Revendications**

1. Appareil (1) pour évaluer la force musculaire des jambes d'un utilisateur, comprenant :

    une unité d'acquisition d'information de force musculaire des jambes (2, 3, 4, 5, 6, 7, 8) ;
    une unité de stockage d'information de référence (9) ; et
    une unité d'évaluation de force musculaire des jambes (2, 3, 4, 5, 6, 7, 8),

dans lequel ladite unité de stockage d'information de référence (9) stocke une information de référence prédéterminée à l'avance,
**caractérisé en ce que** :

    ladite unité d'acquisition d'information de force musculaire des jambes acquiert une information de force musculaire des jambes représentant une masse musculaire des membres inférieurs (LMM) par rapport à au moins un élément pris parmi le poids du corps (Wt) et la taille (Ht) de l'utilisateur,
    ladite unité d'évaluation de force musculaire des jambes évalue la force musculaire des jambes de l'utilisateur en comparant l'information de force musculaire des jambes acquise par ladite unité d'acquisition d'information de force musculaire des jambes (2, 3, 4, 5, 6 7, 8) avec l'information de référence prédéterminée stockée dans ladite unité de stockage d'information de référence ; et
    l'information de référence prédéterminée est une moyenne d'information de force musculaire des jambes pour une pluralité de personnes spécifiques.

2. Appareil (1) pour évaluer la force musculaire des jambes d'un utilisateur selon la revendication 1, dans lequel lesdites personnes spécifiques sont des personnes jeunes qui sont en bonne santé.

3. Appareil (1) pour évaluer la force musculaire des jambes d'un utilisateur selon la revendication 1, dans lequel lesdites personnes spécifiques sont des personnes âgées en tant qu'utilisateur.

4. Appareil (1) pour évaluer la force musculaire des jambes d'un utilisateur selon l'une quelconque des revendications 1 à 3, dans lequel ladite unité d'évaluation de force musculaire des jambes indique la force musculaire des jambes de l'utilisateur en comparant l'information de force musculaire des jambes de l'utilisateur avec ladite information de référence prédéterminée, en supposant que ladite information de référence prédéterminée présente le score de 100 points.

**5.** Appareil (1) pour évaluer la force musculaire des jambes d'un utilisateur selon l'une quelconque des revendications 1 à 4, dans lequel ladite unité d'évaluation de force musculaire des jambes indique la force musculaire des jambes de l'utilisateur en comparant l'information de force musculaire des jambes de l'utilisateur sur un graphique incluant ladite information de référence prédéterminée.

**6.** Appareil (1) pour évaluer la force musculaire des jambes d'un utilisateur selon la revendication 5, dans lequel ladite unité d'évaluation de force musculaire des jambes indique la force musculaire des jambes de l'utilisateur en comparant l'information de force musculaire des jambes de l'utilisateur sur des zones d'un graphique "poids du corps (Wt) ou indice de masse corporelle (BMI) - information de force musculaire des jambes" incluant ladite information de référence prédéterminée.

**7.** Appareil (1) pour évaluer la force musculaire des jambes d'un utilisateur selon la revendication 5, dans lequel il comprend en outre une unité d'acquisition d'information d'âge (4) qui acquiert une information d'âge de l'utilisateur et ladite unité d'évaluation de force musculaire des jambes indique la force musculaire des jambes de l'utilisateur en comparant l'information de force musculaire des jambes de l'utilisateur sur des zones d'un graphique "âge-information de force musculaire des jambes" incluant ladite information de référence prédéterminée.

**8.** Appareil (1) pour évaluer la force musculaire des jambes d'un utilisateur selon l'une quelconque des revendications 5 à 7, dans lequel ladite unité de stockage d'information de référence (9) stocke une seconde information de référence associée à ladite information de référence prédéterminée à l'avance et ladite unité d'évaluation de force musculaire des jambes indique la force musculaire des jambes de l'utilisateur en comparant l'information de force musculaire des jambes de l'utilisateur sur un graphique incluant ladite information de référence prédéterminée et ladite seconde information de référence.

**9.** Appareil (1) pour évaluer la force musculaire des jambes d'un utilisateur selon l'une quelconque des revendications 1 à 8, dans lequel ladite unité d'acquisition d'information de force musculaire des jambes (2, 3, 4, 5, 6, 7, 8, 14) inclut :

une unité d'acquisition d'information de corps (4, 14) ;
une unité de mesure d'impédance bioélectrique (BI) (2, 3, 5, 6, 7) ;
une unité de calcul de masse musculaire des membres inférieurs (LMM) (8) ; et
une unité de calcul d'information de force musculaire des jambes (8), dans lequel ladite unité d'acquisition d'information de corps (9, 14) acquiert une information de corps incluant au moins la taille (Ht) et le poids du corps (Wt) de l'utilisateur,
ladite unité de mesure de BI (2, 3, 5, 6, 7) mesure une impédance bioélectrique entre des parties de corps spécifiques de l'utilisateur,
ladite unité de calcul de masse musculaire des membres inférieurs (LMM) (8) calcule une masse musculaire des membres inférieurs (LMM) sur la base de l'information de corps acquise par ladite unité d'acquisition d'information de corps (4, 14) est sur la base de l'impédance bioélectrique mesurée par ladite unité de mesure de BI (2, 3, 5, 6, 7) ;
ladite unité de calcul d'information de force musculaire des jambes (8) calcule une information de force musculaire des jambes de l'utilisateur sur la base de l'information de corps acquise par ladite unité d'acquisition d'information de corps (4, 14) et sur la base de la masse musculaire des membres inférieurs (LMM) calculée par ladite unité de calcul de masse musculaire des membres inférieurs (LMM) (8).

**10.** Appareil pour évaluer la force musculaire des jambes d'un utilisateur selon la revendication 9, dans lequel ladite unité de calcul d'information de force musculaire des jambes (8) calcule l'information de force musculaire des jambes conformément à une équation "masse musculaire des membres inférieurs (LMM)/poids du corps (Wt)".

**11.** Appareil (1) pour évaluer la force musculaire des jambes d'un utilisateur selon la revendication 9, dans lequel ladite unité de calcul d'information de force musculaire des jambes calcule l'information de force musculaire des jambes conformément à une équation "masse musculaire des membres inférieurs (LMM)/taille$^2$ (Ht$^2$)".

**12.** Appareil (1) pour évaluer la force musculaire des jambes d'un utilisateur selon l'une quelconque des revendications 9 à 11, dans lequel ladite unité d'acquisition d'information de corps (4, 14) acquiert la taille (Ht) de l'utilisateur en l'entrant ou en la mesurant et acquiert le poids du corps (Wt) de l'utilisateur en le mesurant, et ladite unité de mesure de BI (2, 3, 5, 6, 7) mesure l'impédance bioélectrique entre les deux pieds de l'utilisateur.

**13.** Appareil (1) pour évaluer la force musculaire des jambes d'un utilisateur selon l'une quelconque des revendications 9 à 11, dans lequel ladite unité de mesure de BI (2, 3, 5, 6, 7) mesure l'impédance bioélectrique entre les deux mains et les deux pieds de l'utilisateur.

**14.** Appareil (1) pour évaluer la force musculaire des jambes d'un utilisateur selon l'une quelconque des revendications 1 à 13, dans lequel ledit utilisateur sous test est une personne d'âge avancé.

**15.** Support d'enregistrement lisible par ordinateur comportant un programme enregistré dessus pour faire fonctionner un ordinateur en tant qu'appareil (1) pour évaluer la force musculaire des jambes d'un utilisateur selon l'une quelconque des revendications 1 à 14.

**16.** Programme pour faire fonctionner un ordinateur en tant qu'appareil (1) pour évaluer la force musculaire des jambes d'un utilisateur selon l'une quelconque des revendications 1 à 14.

# FIG.1

FIG.2

POWER SUPPLY SWITCHING UNIT — 5

CURRENT SUPPLY UNIT — 6

VOLTAGE MEASUREMENT UNIT — 7

CONTROLLER UNIT — 8

INPUT/OUTPUT CONTROL UNIT — 12

BODY WEIGHT MEASUREMENT UNIT — 14

DISPLAY UNIT — 11

INPUT UNIT — 10

POWER SUPPLY — 13

STORAGE UNIT — 9

4

2a   2b   3a   3b

EP 1 479 340 B1

**FIG.3**

START

S1 — POWER ON AND INITIALIZATION

IS PERSONAL INFORMATION ALREADY REGISTERED?

YES

NO

S2 — ENTER SEX AND AGE

S3 — ENTER HEIGHT

S4 — MEASURE BODY WEIGHT

S5 — MEASURE IMPEDANCE

S6 — CALCULATE PERCENT FAT AND LEG MUSCLE MASS

S7 — CALCULATE LEG STRENGTH INFORMATION AND SCORE FOR LEGS

S8 — DISPLAY BODY WEIGHT AND BODY PERCENT FAT

S9 — DISPLAY SCORE FOR LEGS AND BAR GRAPH

DISPLAY "AGE – SCORE FOR LEGS" GRAPH — S10

DISPLAY "BMI – SCORE FOR LEGS" GRAPH — S11

HAS DISPLAYED THREE TIMES?

NO — TO S8

YES

END — S12

14

## FIG.4

BODY WEIGHT

# 69.1 kg

---------------------------------

PERCENT FAT

# 22.0 %

## FIG.5

SCORE FOR LEGS

# 95 POINTS

- - - - - - - - - - - - - - - - -

| LOW | SLIGHTLY LOW | NORMAL |

## FIG.6

## FIG.7

LOWER LIMBS MUSCLE MASS

# 18.4 kg

LEG STRENGTH AGE

# 54 EQUIVALENT TO YEARS OLD

# FIG.8

**SCORE FOR LEGS**

|  | UNDERWEIGHT | NORMAL | OVERWEIGHT |
|---|---|---|---|
| **NORMAL** | (A) | (B) | (C) ★ |
| **SLIGHTLY LOW** | (D) | (E) | (F) |
| **LOW** | (G) | (H) | (I) |

UNDERWEIGHT  NORMAL  OVERWEIGHT  **BMI**

**EP 1 479 340 B1**

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 1306051 A
- WO 01015600 A